(19) Europäisches Patentamt
European Patent Office
Office européen des brevets

(11) Veröffentlichungsnummer: **0 013 732**
**B1**

# (12) EUROPÄISCHE PATENTSCHRIFT

(45) Veröffentlichungstag der Patentschrift: **18.03.87**

(21) Anmeldenummer: **79105109.7**

(22) Anmeldetag: **12.12.79**

(51) Int. Cl.⁴: **C 07 D 233/84,** C 07 D 405/04, C 07 D 409/04, C 07 D 401/04, A 61 K 31/415, A 61 K 31/44

## (54) Imidazolderivate, Verfahren zu ihrer Herstellung und diese Imidazolderivate enthaltende pharmazeutische Zusammensetzungen.

(30) Priorität: **28.12.78 DE 2856909**

(43) Veröffentlichungstag der Anmeldung: **06.08.80 Patentblatt 80/16**

(45) Bekanntmachung des Hinweises auf die Patenterteilung: **18.03.87 Patentblatt 87/12**

(84) Benannte Vertragsstaaten: **AT BE CH DE FR GB IT LU NL SE**

(56) Entgegenhaltungen:
**EP-A-0 004 648**
**EP-A-0 005 545**
**DE-A-2 635 876**
**DE-A-2 805 166**

**Medicinal Chemistry, 3. Edition, Part I, (Burger A.), 1970, p. 73**

**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Niedballa, Ulrich, Dr., Gosslerstrasse 28 a, D-1000 Berlin 33 (DE)**
Erfinder: **Böttcher, Irmgard Dr., Rodelbahnpfad 5, D-1000 Berlin 28 (DE)**

**EP 0 013 732 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die Erfindung betrifft neue Imidazol-Derivate der allgemeinen Formel I gemäß Anspruch 1, ein Verfahren zu ihrer Herstellung und pharmazeutische Präparate, die diese Imidazol-Derivate als Wirkstoffe enthalten.

Durch Halogenatome substituierte Phenylreste $AR_1$ und $AR_2$ sind beispielsweise Mono- oder Difluorphenylreste oder Mono- oder Dichlorphenylreste und insbesondere para-Fluorphenylreste oder para-Chlorphenylreste. Alkylsubstituierte Phenylreste sind solche, deren Alkylgruppen 1 bis 4 Kohlenstoffatome (beispielsweise Methyl-, Äthyl-, Propyl- oder Isopropylgruppen) enthalten. Durch Alkoxygruppen substituierte Phenylreste sind solche, deren Alkoxygruppen 1 bis 4 Kohlenstoffatome (Methoxy-, Äthoxy-, Propyloxy- oder Isopropyloxygruppen) enthalten. Unter einer durch Dialkylaminogruppen substituierten Phenylgruppe soll eine Gruppe verstanden werden, deren Dialkylaminorest 2 bis 6 Kohlenstoffatome hat, so zum Beispiel die Dimethylaminogruppe, die Methyläthylaminogruppe oder die Diäthylaminogruppe.

Die Substituenten $AR_1$ und $AR_2$ können gleich oder verschieden sein, mit der Maßgabe, daß nicht beide Substituenten einen unsubstituierten Phenylrest darstellen.

Vorzugsweise bedeutet der Substituent $R_1$ eine unsubstituierte oder in der 2-Position durch eine Hydroxygruppe, eine Alkoxygruppe mit 1 bis 4 Kohlenstoffatomen (zum Beispiel eine Methoxygruppe, eine Äthoxygruppe, eine Propyloxygruppe oder eine Isopropyloxygruppe) oder eine Acyloxygruppe mit 1 bis 6 Kohlenstoffatomen (zum Beispiel eine Formyloxygruppe, eine Aretoxygruppe, eine Propionyloxygruppe oder eine Butyryloxygruppe) substituierte Alkylgruppe. Insbesondere soll unter dem Substituenten $R_1$ ein Wasserstoffatom oder eine Methylgruppe verstanden werden. Als durch eine 3 bis 6 Kohlenstoffatome enthaltende Cycloalkylgruppe substituierte Alkylgruppe mit 1 bis 4 Kohlenstoffatome seien beispielsweise genannt: die Cyanomethylgruppe, die Benzylgruppe, die Cyclopropylmethylgruppe, die Cyclobutylmethylgruppe, die Cyclopentylmethylgruppe, die Cyclohexylmethylgruppe.

Die Imidazol-Derivate der allgemeinen Formel I gemäß Anspruch 1 zeichnen sich durch eine ausgeprägte antiinflammatorische, antiallergische und immunstimmulierende Wirksamkeit aus.

Darüberhinaus zeichnen sich die Imidazol-Derivate der allgemeinen Formel I dadurch aus, daß sie eine sehr günstige Dissoziation zwischen erwünschter pharmakologischer Wirksamkeit und unerwünschter - insbesondere ulcerogenen - Nebenwirkungen besitzen. Diese Dissoziation ist besonders ausgeprägt, bei solchen Imidazol-Derivaten der allgemeinen Formel I in denen n die Bedeutung der Ziffern 1 oder 2 hat.

Somit eignen sich die neuen Verbindungen in Kombination mit den in der galenischen Pharmazie üblichen Trägermitteln zur Behandlung zum Beispiel von akuter und chronischer Polyarthritis, Neurodermitis, Asthma bronchiale und Heufieber.

Die Herstellung der Arzneimittelspezialitäten erfolgt in üblicher Weise, indem man die Wirkstoffe mit geeigneten Zusätzen, Trägersubstanzen und Geschmackskorriegentien in die gewünschte Applikationsform wie Tabletten, Drageee, Kapseln, Lösungen und Inhalationsmitteln überführt.

Für die orale Anwendung eignen sich insbesondere Tabletten, Dragees und Kapseln, welche beispielsweise 1 bis 250 mg Wirkstoff und 50 mg bis 2 g pharmakologisch unwirksamen Trägers, wie zum Beispiel Laktose, Amylose, Talkum, Gelatine und Magnesiumstearat, sowie die üblichen Zusätze enthalten.

Die neuen Imidazol-Derivate der allgemeinen Formel I gemäß Anspruch 1 können nach an sich bekannten Verfahren hergestellt werden. Ein geeignetes Herstellungsverfahren ist beispielsweise die im Anspruch 29 beschriebene Synthese, die unter den bekannten Bedingungen durchgeführt werden kann (Liebigs Annalen 284, 1894, Seite 9, f., J.Chem. Soc. 1931, Seite 3043 f., J. Med.Chem. 20, 1977, Seite 563 f., Liebigs Annalen 214, 1882, Seite 257, f., J. Chem.Soc. 1942, Seite 232 f., J.Chem. Soc. 1963, Seite 2195 f., Houben Weyl: Methoden der organischen Chemie Band IX, Seite 229 f., Bull Soc. France 1977, Seite 271 f., Deutsche Offenlegungsschrift 26 35 876, J.Chem.Soc., 1963, 2185, J. Amer.Chem.Soc., 100, 1978, 1481 und Chem. Ber. 111, 1978, 2785).

Nach erfolgter Synthese können die racemischen Imidazol-Derivate der allgemeinen Formel I gemäß Anspruch 1 in an sich bekannter Weise in ihre optischen Antipoden gespalten werden, beispielsweise indem man diese durch Säulenchromatographie an optisch aktiven Trägern (z.B. Sephadex(R)) chromatographiert.

Die Ausgangsverbindungen für das erfindungsgemäße Verfahren sind bekannt oder können in an sich bekannter Weise hergestellt werden (Synthesis 1976 Seite 733 f. und Zhur. Obsch. Khim 31, 1961, Seite 1039 f., Houben-Weyl, Band IX, 1955, 59). Nachfolgend ist das Herstellungsverfahren für diese Ausgangsverbindungen an Hand einiger typischer Verbindungen beschrieben:

Zu einer Lösung von 20,43 g 4-Dimethylaminobenzoin in 250 ml Dimethylformamid werden 12,18 g Ammoniumthiocyanat gegeben, und man erwarmt 14 Stunden auf 80°. Nach dem Abkühlen rührt man die Lösung in Eiswasser ein, saugt die ausgefallenen Kristalle ab und kristallisiert sie aus heißem Äthanol um. Man erhält so 14,62 g 4-(4-Dimethyl-amino)-5-phenyl-2-mercapto-imidazol vom Schmelzpunkt 277-280°C.

Die nachfolgenden Beispiele dienen zur Erläuterung des erfindungsgemäßen Verfahrens.

**Beispiel 1**

Ein Gemisch aus 3,12 g 4,5-Bis-(4-methoxyphenyl)-2-mercaptoimidazol, 1,1 g Chlormethylcyclopropan und 100 ml Äthanol wird 4 Stunden unter Argon am Rückfluß erhitzt. Nach dem Abkühlen neutralisiert man mit 2 N Natronlauge, gießt in 500 ml Wasser, nimmt den ausgeschiedenen Feststoff in Methylenchlorid auf, trocknet

die organische Lösung über Natriumsulfat und engt sie im Vakuum zur Trockne ein. Man kristallisiert den Rückstand aus Dibutyläther und erhält so 2,91 g 4,5-Bis-(4-methyloxyphenyl)-2-(cyclopropylmethylthio)-imidazol vom Schmelzpunkt 119° C.

**Beispiel 2**

Zu einer Lösung von 1,83 g 4,5-Bis-(4-methoxyphenyl)-2-(cyclopropylmethylthio)-imidazol in 200 ml Metbylenchlorid werden 1,082 g 3-Chlorperbenzoesäure (80%) in 100 ml Methylenchlorid getropft. Man läßt 2 Stunden nachrühren, wäscht mit gesättigter Natriumbikarbonatlösung, trocknet die organische Lösung über Natriumsulfat und engt sie im Vakuum zur Trockene ein. Der Rückstand wird aus Dibutyläther kristallisiert. Man erhält so 1,41 g 4,5-Bis-(4methoxyphenyl)-2-(cyclo-propylmethylsulfinyl)-imidazol vom Schmelzpunkt 147° C.

**Beispiel 3**

Zu einer Lösung von 1,83 g 4,5-Bis-(4-methoxyphenyl)-2-(cyclopropylmethylthio)-imidazol in 200 ml Methylenchlorid werden 2,164 g 3-Chlorperbenzoesäure (80%) in 200 ml Methylenchlorid getropft. Man läßt 2 Stunden nachrühren, wäscht mit gesättigter Natriumbikarbonatlösung, trocknet die organische Lösung über Natriumsulfat und engt sie im Vakuum zur Trockene ein. Der Rückstand wird aus Dibutyläther/Äthanol kristallisiert. Man erhält so 1,43 g 4,5-Bis-(4-methoxyphenyl)-2-(cyclo-propylmethylsulfonyl)-imidazol vom Schmelzpunkt 149° C.

**Beispiel 4**

Zu einer Lösung von 1,86 g Brommethylcyclopentan in 150 ml Äthanol gibt man 6,25 g 4,5-Bis-(4-methoxyphenyl)-2-mercaptoimidazol und erhitzt 8 Stunden unter Argon am Rückfluß. Nach dem Abkühlen neutralisiert man mit 2 N Natronlauge, gießt in Wasser, nimmt den ausgeschiedenen Feststoff in Methylenchlorid auf, trocknet die organische Lösung über Natriumsulfat und engt sie im Vakuum zur Trockene ein. Der Rückstand wird aus Methylenchlorid/Hexan kristallisiert und aus Äthanol umkristallisiert. Man erhält so 6,57 g 4,5-Bis-(4-methoxyphenyl)-2-(cyclopentylmethylthio)-imidazol vom Schmelzpunkt 175-77° C.

**Beispiel 5**

Zu einer Lösung von 3,94 g 4,5-Bis-(4-methoxyphenyl)-2-(cyclopentylmethylthio)-imidazol in 150 ml Methylenchlorid werden 2,164 g 2-Chlorperbenzoesäure (80%) in 200 ml Methylenchlorid getropft. Man läßt 2 Stunden nachrüren, wäscht mit gesättigter Natriumbikarbonatlösung, trocknet die organische Lösung über Natriumsulfat und engt sie im Vakuum zur Trockene ein. Der Rückstand wird aus Methylenchlorid/Hexan kristallisiert.

Man erhält so 3,63 g 4,5-Bis-(4-methoxyphenyl)-2-(cyclopentylmethylsulfinyl)-imidazol vom Schmelzpunkt 212-13° C.

**Beispiel 6**

Zu einer Lösung von 3,94 g 4,5-Bis-(4-methoxyphenyl)-2-(cyclopentylthio)-imidazol in 150 ml Methylenchlorid werden 4,328 g 3-Chlorperbenzoesäure (80%) in 400 ml Methylenchlorid anteilweise gegeben. Man läßt 2 Stunden nachrühren, wäscht mit gesättigter Natriumbikarbonatlösung, trocknet die organische Lösung über Natriumsulfat und engt sie im Vakuum zur Trockene ein. Der Rückstand wird aus Methylenchlorid/Hexan kristallisiert. Man erhält so 3,53 g 4,5-Bis-(4-methoxyphenyl)-2-(cyclopentylmethylsulfonyl)-imidazol vom Schmelzpunkt 180-81° C.

**Beispiel 7**

Zu einer Lösung von 150 ml Äthanol und 4,37 g Bromcyclopentan gibt man 6,25 g 4,5-Bis-(4-methoxyphenyl)-2-mercaptoimidazol und erhitzt 12 Stunden unter Argon am Rückfluß. Nach dem Abkühlen neutralisiert man mit

2 N Natronlauge, gießt in 700 ml Wasser,̇ nimmt den ausgeschiedenen Feststoff in Methylenchlorid auf, trocknet die organische Lösung über Natriumsulfat und engt sie im Vakuum zur Trockene ein. Der amorphe Rückstand wird in Äther gelöst und nach Zusatz von Hexan zur Kristallisation gebracht. Nach Umkristallisieren aus Äther/Hexan erhält man 6,08 g 4,5-Bis-(4-methyloxyphenyl)-2-(cyclo-pentylthio)-imidazol vom Schmelzpunkt 169-70° C.

**Beispiel 8**

Zu einer Lösung von 953 mg 4,5-Bis-(4-methoxyphenyl)-2-(cyclopentylthio)-imidazol in 150 ml Methylenchlorid werden 541 mg 3-Chlorperbenzoesäure (80%) in 70 ml Methylenchlorid anteilweise gegeben. Man läßt 15 Minuten nachrühren, wäscht mit gesättigter Natriumbikarbonatlösung, trocknet die organische lösung über Natriumsulfat und engt sie im Vakuum zur Trockene ein. Der Rückstand wird aus Methylenchlorid/Hexan kristallisiert. Man erhält so 920 mg 4,5-Bis-(4-methoxyphenyl)-2-(cyclopentyl-sulfinyl)-imidazol als farblose Nadeln vom Schmelzpunkt 159-60° C.

**Beispiel 9**

Zu einer Lösung von 953 mg 4,5-Bis-(4-methoxyphenyl)-2-(cyclopentylthio)-imidazol in 150 ml Methylenchlorid werden 1082 mg 3-Chlorperbenzoesäure (80%) in 100 ml Methylenchlorid anteilweise gegeben. Män läßt 15 Minuten nachrühren, wäscht mit gesättigter Natriumbikarbonatlösung, trocknet die organische Lösung über Natriumsulfat und engt sie im Vakuum zur Trockene ein. Der Rückstand wird aus Methylenchlorid/Hexan kristallisiert. Man erhält so 800 mg 4,5-Bis-(4-methoxyphenyl)-2-(cyclopentylsulfonyl)-imidazol als rosa Nadeln vom Schmelzpunkt 152-53° C.

**Beispiel 10**

3,12 g 4,5-Bis(4-methoxyphenyl)-2-mercaptoimidazol werden in 100 ml einer 0,1 N Natriumäthylatlösung gelöst, mit 1,1 g Cyclopentenoxid versetzt und 12 Stunden unter Argon am Rückfluß erhitzt. Nach dem Abkühlen gießt man in 500 ml Wasser, nimmt den ausgefallenen Feststoff in Methylenchlorid auf, trocknet die organische Lösung über Natriumsulfat und engt sie im Vakuum zur Trockene ein. Der Rückstand wird aus Cyclohexan/ Äthanol kristallisiert, und man erhält so 3,19 g 4,5-Bis-(4-methoxyphenyl)-2-(2-hydroxy-cyclopentylthio)-imidazol vom Schmelzpunkt 103-105° C.

**Beispiel 11**

Zu einer Lösung von 1,98 g 4,5-Bis(4-methoxyphenyl)-2-(2-hydroxycylopentylthio)-imidazol in 200 ml Methylenchlorid werden 1,082 g 3-Chlorperbenzoesäure (80%) in 100 ml Methylenchlorid getropft. Man läßt 2 Stunden nachrühren, wäscht mit gesättigter Natriumbikarbonatlösung, trocknet die organische Lösung über Natriumsulfat und eng sie im Vakuum zur Trockene ein. Der Rückstand wird aus Methylenchlorid/Hexan kristallisiert. Man erhält so 1,66 g 4,5-Bis-(4-methoxyphenyl)-(2-hydroxycyclopentylsulfinyl)-imidazol vom Schmelzpunkt 194-96° C.

**Beispiel 12**

Zu einer Lösung von 1,98 g 4,5-Bis(4-methoxyphenyl)-2-(2-hydroxycyclopentylthio)-imidazol in 200 ml Methylenchlorid werden 2,164 g 3-Chlorperbenzoesäure (80%) in 200 ml Methylenchlorid getropft. Man läßt 2 Stunden nachrühren, wäscht mit gesättigter Natriumbikarbonatlösung, trocknet die organische Lösung über Natriumsulfat und eng sie im Vakuum zur Trockene ein. Der Rückstand wird aus Methylenchlorid/Hexan kristallisiert. Man erhält so 1,73 g 4,5-Bis-(4-methoxyphenyl)-2-(2-hydroxycyclopentylsulfonyl)-imidazol vom Schmelzpunkt 176-77° C.

**Beispiel 13**

Zu einer Lösung von 12,5 g 4,5-Bis-(4-methoxyphenyl)-2-mercaptoimidazol in 800 ml Dioxan/Methylenchlorid 3:1 werden 4,50 ml Perhydrol gegeben. Man läßt 4 Stunden bei Raumtemperatur rühren, engt dann die Lösung im Vakuum auf 100 ml ein und läßt das gebildete Produkt aus der Lösung kristallisieren. Man erhält so 9,66 g Bis-[4,5-bis-(4-methoxyphenyl)-2-imidazolyl]-disulfid vom Schmelzpunkt 249°C.

Zu einer Lösung von 9,33 g Bis-[4,5-bis-(4-methoxyphenyl)-2-imidazolyl]-disulfid in 100 ml absolutem Hexamethylphosphorsäuretriamid werden 1,584 g Natriumhydrid (50%, in Weißöl) gegeben. Man läßt 30 Minuten bei Raumtemperatur rühren, versetzt dann mit 3,42 g Trimethylchlorsilan und läßt weitere 3 Stunden bei Raumtemperatur rühren. Dann deckt mit Argon ab und läßt 30 ml einer ca. 0,9 N Cyclopropyllithium-Lösung in Äther unter Rühren zutropfen. Nach beendeter Zugabe läßt man noch 4 Stunden bei 60°C nachrühren. Die erhaltene Lösung wird in 600 ml Eiswasser gegossen, und man extrahiert mit Äther. Die organische Lösung wird mit Wasser gewaschen, über Natriumsulfat getrocknet und im Vakuum zur Trockene eingeengt. Der Rückstand wird durch Säulenchromatographie an 300 g Kieselgel mit Cyclohexan/Essigester 1:1 gereinigt. Man erhält so 1,93 g 4,5-Bis-(4-methoxyphenyl)-2-(cyclopropylthio)-imidazol, das beim Abziehen des Lösungsmittels als amorpher Schaum anfällt.

$C_{20}H_{20}N_2O_2S$ (352,458)
Ber.: 68,16% C 5,72% H 7,95% N 9,10% S
Gef.: 68,04 5,82 7,91 9,01

Aus Äther/Hexan erhält man Kristalle, die bei 135°C schmelzen.

**Beispiel 14**

Unter den Bedingungen des Beispiel 2 werden 1,76 g 4,5-Bis -(4-methoxyphenyl)-2-(cyclopropylthio)-imidazol oxydiert, und man erhält nach Kristallisation aus Äther 1,53 g 4,5-Bis-(4-methoxyphenyl)-2-(cyclopropylsulfinyl)-imidazol vom Schmelzpunkt 180°C.

**Beispiel 15**

Unter den Bedingungen des Beispiels 3 werden 1,76 g 4,5-Bis-(4-methoxyphenyl)-2-(cyclopropylthio)-imidazol oxydiert, und man erhält nach Kristallisation aus Dibutyläther 1,52 g 4,5-Bis-(4-methoxyphenyl)-2-(cyclopropylsulfonyl)-imidazol vom Schmelzpunkt 176°C.

**Beispiel 16**

Ein Gemisch aus 2,88 g 4,5-Bis-(4-fluorphenyl)-2-mercaptoimidazol und 1,1 g Chlormethylcyclopropan in 100 ml absolutem Äthanol wird 4 Stunden unter Argon am Rückfluß erhitzt. Man neutralisiert die abgekühlte Lösung mit 2 N Natronlauge, gießt sie in 500 ml Wasser, saugt das ausgefallene Produkt ab, wäscht es mit Wasser und trocknet es im Vakuum bei 60°. Man erhält so 2,67 g 4.5-Bis-(4-fluorphenyl)-2-(cyclopropylmethylthio)-imidazol vom Schmelzpunkt 210-211°C.

**Beispiel 17**

Unter den Bedingungen des Beispiels 2 werden 1,71 g 4,5-Bis-(4-fluorphenyl)-2-(cyclopropylmethylthio)-imidazol oxydiert, und man erhält nach Abziehen des Lösungsmittels 1,34 g 4,5-Bis-(4-fluorphenyl)-2-(cyclopropylmethylsulfinyl)-imidazol vom Schmelzpunkt 181-183°C (Methylenchlorid/Hexan).

**Beispiel 18**

Unter den Bedingungen des Beispiels 3 werden 1,71 g 4,5-Bis-(4-fluorphenyl)-2-(cyclopropylmethylthio)-imidazol oxydiert, und man erhält nach Abziehen des Lösungsmittels 1,47 g 4,5-Bis-(4-fluorphenyl)-2-(cyclopropylmethylsulfonyl)-imidazol vom Schmelzpunkt 251°C (Methylenchlorid/Hexan).

**Beispiel 19**

In einer Lösung von 300 mg Natrium in 150 ml absolutem Äthanol werden 3,51 g 4,5:Bis-(4-methoxyphenyl)-2-(cyclopentylthio)-imidazol unter Rühren und Abdeckung mit Argon gelöst. Dann gibt man 1,85 g Methyljodid hinzu und erhitzt über Nacht am Rückfluß. Nach dem Abkühlen gießt man in 600 ml Wasser, nimmt das ausgefallene Produkt in Methylenchlorid auf, trocknet die organische Lösung über Natriumsulfat und engt sie im Vakuum zur Trockne ein. Der Rückstand wird aus Methanol kristallisiert, und man erhält 2,49 g 4,5-Bis-(4-methoxyphenyl)-1-methyl-2-(cyclopentylthio)-imidazol vom Schmelzpunkt 122-124° C.

**Patentansprüche**

1. Imidazol-Derivate der allgemeinen Formel I

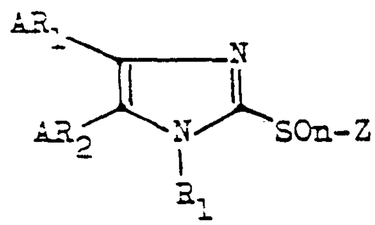

(I),

worin
$AR_1$ und $AR_2$ einen gegebenenfalls durch Halogenatome, 1 bis 4 Kohlenstoffatome enthaltende Alkylgruppen, 1 bis 4 Kohlenstoffatome enthaltende Alkoxygruppen oder 2 bis 6 Kohlenstoffatome enthaltende Dialkylaminogruppen substituierten Phenylrest bedeuten, mit der Maßgabe, daß nicht beide Substituenten $AR_1$ und $AR_2$ einen unsubstituierten Phenylrest darstellen, $R_1$ ein Wasserstoffatom, oder einen gegebenenfalls durch Hydroxygruppen, Alkoxygruppen mit 1 bis 4 Kohlenstoffatomen oder Acyloxygruppen mit 1 bis 6 Kohlenstoffatomen substituierter Alkylrest mit 1 bis 4 Kohlenstoffatomen bedeutet, n die Ziffern 0, 1 oder 2 darstellt und Z einen gegebenenfalls durch eine Hydroxygruppe substituierten Cycloalkylrest mit 3 bis 8 Kohlenstoffatomen oder eine durch eine 3 bis 6 Kohlenstoffatome enthaltende Cycloalkylgruppe substituierte Alkylgruppe mit 1 bis 4 Kohlenstoffatomen bedeuten, und deren Salze mit physiologisch unbedenklichen Säuren.

2. Imidazol-Derivate der allgemeinen Formel 1 gemäß Anspruch 1, dadurch gekennzeichnet, daß die Substituenten $AR_1$ und $AR_2$ einen, gegebenenfalls in para-Stellung durch ein Fluoratom, ein Chloratom, eine 1 bis 4 Kohlenstoffatome enthaltende Alkylgruppe, eine 1 bis 4 Kohlenstoffatome enthaltende Alkoxygruppe oder eine 2 bis 6 Kohlenstoffatome enthaltende Dialkylaminogruppe substituierten Phenylrest, darstellen.

3. Imidazol-Derivate der allgemeinen Formel 1 gemäß Anspruch 2, dadurch gekennzeichnet, daß die Substituenten $AR_1$ und $AR_2$ einen Phenylrest, einen 4-Fluorphenylrest, einen 4-Chlor-phenylrest, einen 4-Methylphenylrest, einen 4-Methoxyphenyl-rest, oder einen 4-Dimethylaminophenylrest, darstellen.

4. Imidazol-Derivate der allgemeinen Formel 1 gemäß Anspruch 1 bis 3, dadurch gekennzeichnet, daß der Substituent $R_1$ ein Wasserstoffatom, eine 1 bis 4 Kohlenstoffatome enthaltende Alkylgruppe, eine 2-Hydroxyäthylengruppe oder eine 2-Acyloxyäthylengruppe mit 1 bis 6 Kohlenstoffatomen im Acyloxyrest bedeutet.

5. Imidazol-Derivate der allgemeinen Formel 1 gemäß Anspruch 1 bis 4, dadurch gekennzeichnet, daß n die Ziffern 1 oder 2 bedeutet.

6. Imidazol-Derivate der allgemeinen Formel 1 gemäß Anspruch 1 bis 5, dadurch gekennzeichnet, daß der Substituent Z einen 3 bis 7 Kohlenstoffatome enthaltenden Cycloalkylrest darstellt

7. Imidazol-Derivate der allgemeinen Formel 1 gemäß Anspruch 1, dadurch gekennzeichnet, daß der Substituent Z eine durch eine 3 bis 6 Kohlenstoffatome enthaltende Cycloalkylgruppe substituierte Methylgruppe darstellt.

8. 4,5-Bis-(4-methoxyphenyl)-2-(cyclopropylmethylthio)-imidazol.

9. 4,5-Bis-(4-methoxyphenyl)-2-(cyclopropylmethylsulfinyl)-imidazol.

10. 4 5-Bis-(4-methoxyphenyl)-2-(cyclopropylmethylsulfonyl)-imidazol.

11. 4,5-Bis-(4-methoxyphenyl)-2-(cyclopentylmethylthio)-imidazol.

12. 4,5-Bis-(4-methoxyphenyl)-2-(cyclopentylmethylsulfinyl)-imidazol.

13. 4,5-Bis-(4-methoxyphenyl)-2-(cyclopentylmethylsulfonyl)-imidazol.

14. 4,5-Bis-(4-methoxyphenyl)-2-(cyclopentylthio)-imidazol.

15. 4,5-Bis-(4-methoxyphenyl)-2-(cyclopentylsulfinyl)-imidazol.

16. 4,5-Bis-(4-methoxyphenyl)-2-(cyclopentylsulfonyl)-imidazol.
17. 4,5-Bis-(4-methoxyphenyl)-2-(2-hydroxycyclopentylthio)-imidazol.
18. 4,5-Bis-(4-methoxyphenyl)-2-(2-hydroxycyclopentylsulfinyl)-imidazol.
19. 4,5-Bis-(4-methoxyphenyl)-2-(2-hydroxycyclopentylsulfonyl)-imidazol.
20. 4,5-Bis-(4-methoxyphenyl)-2-(cyclopropylthio)-imidazol.
21. 4,5-Bis-(4-methopyphenyl)-2-(cyclopropylsulfinyl)-imidazol,
22. 4,5-Bis-(4-methoxyphenyl)-2-(cyclopropylsulfonyl)-imidazol.
23. 4,5-Bis-(4-fluorphenyl)-2-(cyclopropylmethylthio)-imidazol.
24. 4,5-Bis-(4-fluorphenyl)-2-(cyclopropylmethylsulfinyl)-imidazol.
25. 4,5-Bis-(4-fluorphenyl)-2-(cyclopropylmethylsulfonyl)-imidazol.
26. 4,5-Bis-(2-methoxyphenyl)-1-methyl-2-(cyclopentylthio)-imidazol.
27. Pharmazeutische Präparate, gekennzeichnet durch ein oder zwei Imidazol-Derivate gemäß Anspruch 1 bis 26.
28. Pharmazeutische Präparate gemäß Anspruch 27 zur Behandlung entzündlicher oder allergischer Erkrankungen des Menschen.
29. Verfahren zur Herstellung von Imidazol-Derivaten der allgemeinen Formel 1 gemäß Anspruch 1, dadurch gekennzeichnet, daß man in an sich bekannter Weise

a) ein Imidazol-Derivat der allgemeinen Formel II

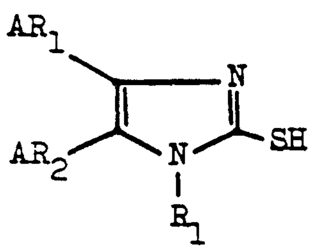

(II),

worin $AR_1$, $AR_2$ und $R_1$ die obengenannte Bedeutung besitzen, mit einer Verbindung der allgemeinen Formel III

W Z (III),

worin Z die obengenannte Bedeutung besitzt und W ein Halogenatom, einen Alkylsulfonyloxyrest oder einen Arylsulfonyloxyrest darstellt, kondensiert, oder

b) zur Herstellung von Imidazol-Derivaten der allgemeinen Formel I mit Z in der Bedeutung einer Cycloalkylgruppe, ein Disulfid der allgemeinen Formel VII

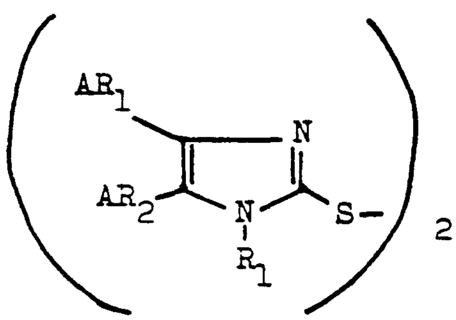

(VII),

mit einem Lithiumcycloalkyl umsetzt, gegebenenfalls die gemäß Verfahrensvariante a bis b erhaltenen in 1-Stellung unsubstituierte Imidazol-Derivate alkyliert, Hydroxylgruppen enthaltende Imidazol-Derivate verestert, vorhandene Thiogruppen zu Sulfinylgruppen oder Sulfonylgruppen oxydiert, und/oder Imidazol-Derivate der allgemeinen Formel I mit physiologisch unbedenklichen Säuren in ihre Salze Oberführt.

**Claims**

1. Imidazole derivatives of general formula I

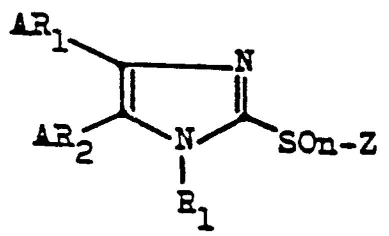

(I)

wherein

$AR_1$ and $AR_2$ are phenyl groups, optionally substituted by halogen atoms, alkyl groups containing 1 to 4 carbon atoms, alkoxy groups containing 1 to 4 carbon atoms or dialkylamino groups containing 2 to 6 carbon atoms, with the proviso that both substituents $AR_1$ and $AR_2$ cannot be an unsubstituted phenyl group, $R_1$ is a hydrogen atom, or an alkyl group of 1 to 4 carbon atoms, optionally substituted by hydroxy groups, alkoxy groups of 1 to 4 carbon atoms or acylalkoxy groups of 1 to 6 carbon atoms, n is the integer 0, 1 or 2 and Z is a cycloalkyl group of 3 to 6 carbon atoms, optionally substituted by a hydroxy group or an alkyl group of 1 to 4 carbon atoms substituted by a cycloalkyl group containing 3 to 6 carbon atoms, and salts of these with physiologically acceptable acids.

2. Imidazole derivatives of general formula 1, according to claim 1, characterised in that the substituents $AR_1$ and $AR_2$ are each a phenyl group, optionally substituted in the para-position by a fluorine atom, a chlorine atom, an alkyl group containing 1 to 4 carbon atoms, an alkoxy group containing 1 to 4 carbon atoms or a dialkylamino group containing 2 to 6 carbon atoms.

3. Imidazole derivatives of general formula I, according to claim 2, characterised in that the substituents $AR_1$ and $AR_2$ are each a phenyl group, a 4-fluorophenyl group, a 4-chlorophenyl group, a 4-methylphenyl group, a 4-methoxyphenyl or a 4-dimethylaminophenyl group.

4. Imidazole derivatives of general formula I, according to claims 1 to 3, characterised in that the substituent $R_1$ is a hydrogen atom, an alkyl group containing 1 to 4 carbon atoms, a 2-hydroxyethyl group or a 2-acyloxyethyl group with 1 to 6 carbon atoms in the acyloxy group.

5. Imidazole derivatives of general formula I. according to claims 1 to 4, characterised in that n is the integer 1 or 2.

6. Imidazole derivatives of general formula 1, according to claims 1 to 5, characterised in that the substituent Z is a cycloalkyl group containing 3 to 7 carbon atoms.

7. Imidazole derivatives of general formula I. according to claims 1, characterised in that the substituent Z is a methyl group, substituted by a cycloalkyl group containing 3 to 6 carbon atoms.

8. 4,5-Bis-(4-methoxyphenyl)-2-(cyclopropylmethylthio)-imidazole.

9. 4,5-Bis-(4-methoxyphenyl)-2-(cyclopropylmethyl-sulphinyl)imidazole.

10. 4,5-Bis-(4-methoxyphenyl)-2-(cyclopropylmethyl-sulphonyl)imidazole.

11. 4,5-Bis-(4-methoxyphenyl)-2-(cyclopentylmethylthio)-imidazole.

12. 4,5-Bis-(4-methoxyphenyl)-2-(cyclopentylmethyl-sulphinyl)imidazole.

13. 4,5-Bis-(4-methoxyphenyl)-2-(cyclopentylmethyl-sulphonyl)imidazole.

14. 4,5-Bis-(4-methoxyphenyl)-2-(cyclopentylthio)-imidazole.

15. 4,5-Bis-(4-methoxyphenyl)-2-(cyclopentylsulphinyl)-imidazole.

16. 4, 5-Bis-(4-methoxyphenyl)-2-(cyclopentylsulphonyl)-imidazole.

17. 4,5-Bis-(4-methoxyphenyl)-2-(2-hydroxycyclopentylthio)-imidazole.

18. 4,5-Bis-(4-methoxyphenyl)-2-(2-hydroxycyclopentyl-sulphinyl)imidazole.

19. 4,5-Bis-(4-methoxyphenyl)-2-(2-hydroxycyclopentyl-sulphonyl)imidazole.

20. 4,5-Bis-(4-methoxyphenyl)-2-(cyclopropylthio)-imidazole.

21. 4,5-Bis- (4-methoxyphenyl) -2- (cyclopropylsulphinyl)-imidazole.

22. 4,5-Bis-(4-methoxyphenyl)-2-(cyclopropylsulphonyl)-imidazole.

23. 4,5-Bis-(4-fluorophenyl)-2-(cyclopropylmethylthio)-imidazole.

24. 4,5-Bis-(4-fluorophenyl)-2-(cyclopropylmethylsulphinyl)-imidazole.

25. 4,5-Bis-(4-fluorophenyl)-2-(cyclopropylmethylsulphonyl)-imidazole.

26. 4,5-Bis-(2-methoxyphenyl)-1-methyl-2-(cyclopentylthio)-imidazole.

27. Pharmaceutical preparations characterised by one or two imidazole derivatives according to claims 1 to 26 in admixture with conventional pharmaceutical carriers.

28. Pharmaceutical preparations according to claim 27 for the treatment of inflammatory or allergic diseases of humans.

29. Process for the preparation of imidazole derivatives of general formula 1, according to claim 1, characterised in that, in a known manner,

a) an imidazole derivative of general formula II

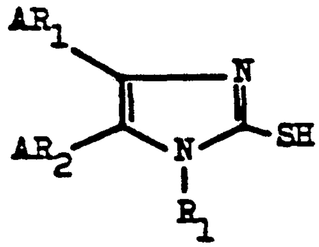

(II)

in which $AR_1$, $AR_2$ and $R_1$ have the meanings given above, is condensed with a compound of general formula III

W-Z III

in which Z has the meaning given above and W is halogen atom, an alkylsulphonyloxy group or an arylsulphonyloxy group, or

b) for the preparation of imidazole derivatives of general formula 1 where Z is a cycloalkyl group, a disulphide of general formula VII

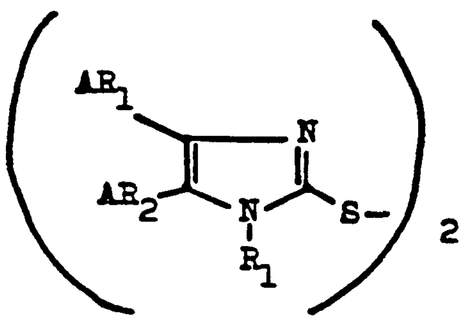

(VII)

is treated with a lithiumcycloalkyl, optionally the imidazole obtained from process variants a to b, which is unsubstituted in the 1-position, is alkylated, imidazole derivatives containing hydroxy groups are esterified, thio groups present are oxidised to the corresponding sulphinyl or sulphonyl groups and/or imidazole derivatives of general formula 1 are converted into their salts with physiologically acceptable acids.

**Revendications**

1. Dérivés de l'imidazole qui répondent à la formule générale I:

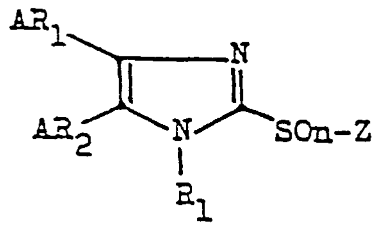

(I)

dans laquelle:

$AR_1$ et $AR_2$ représentent chacun un radical phényle éventuellement porteur d'atomes d'halogènes, de radicaux alkyles contenant de 1 à 4 atomes de carbone, de radicaux alcoxy contenant de 1 à 4 atomes de carbone ou de radicaux dialkylamino contenant de 2 à 6 atomes de carbone, avec la restriction que les deux substituants $AR_1$ et $AR_2$ ne représentent pas chacun en même temps un radical phényle non substitué,

$R_1$ représente un atome d'hydrogène ou un radical alkyle contenant de 1 à 4 atomes de carbone et éventuellement porteur de radicaux hydroxy, de radicaux alcoxy contenant de 1 à 4 atomes de carbone ou de radicaux acyloxy contenant de 1 à 6 atomes de carbone,

n représente un nombre égal à 0, à 1 ou à 2 et

Z représente un radical cycloalkyle contenant de 3 à 8 atomes de carbone et éventuellement porteur d'un radical hydroxy, ou un radical alkyle contenant de 1 à 4 atomes de carbone et portant un radical cycloalkyle contenant de 3 à 6 atomes de carbone, et sels qu'ils forment avec des acides acceptables du point de vue physiologique.

2. Dérivés de l'imidazole de formule générale I selon la revendication 1, caractérisés en ce que les substituants $AR_1$ et $AR_2$ représentent chacun un radical phényle qui porte éventuellement, en position para, un atome de fluor, un atome de chlore, un radical alkyle contenant de 1 à 4 atomes de carbone, un radical alcoxy contenant de 1 à 4 atomes de carbone ou un radical dialkylamino contenant de 2 à 6 atomes de carbone.

3. Dérivés de l'imidazole de formule générale selon ls revendication 2, caractérisés en ce que les substituants $AR_1$ et $AR_2$ représentent chacun un radical phényle, un radical fluoro-4 phényle, un radical chloro-4 phényle, un radical méthyl-4 phényle, un radical méthoxy-4 phényle ou un radical diméthylamino-4 phényle.

4. Dérivés de l'imidazole de formule générale I selon l'une quelconque des revendicstions 1 à 3, caractérisés en ce que $R_1$ représente un atome d'hydrogène, un radical alkyle contenant de 1 à 4 atomes de carbone, un radical hydroxy-2 éthyle ou un radical acyloxy-2 éthyle dont le radical acyloxy contient de 1 à 6 atomes de carbone.

5. Dérivés de l'imidazole de formule générale I selon l'une quelconque des revendications 1 à 4, caractérisés en ce que n désigne le nombre 1 ou le nombre 2.

6. Dérivés de l'imidazole de formule générale I selon l'une quelconque des revendications 1 à 5, caractérisés en ce que le substituant Z est un radical cycloalkyle contenant de 3 à 7 atomes de carbone.

7. Dérivés de l'imidazole de formule générale I selon la revendication 1, caractérisés en ce que le substituant Z est un radical méthyle porteur d'un radical cycloalkyle contenant de 3 à 6 atomes de carbone.

8. Le bis-(méthoxy-4 phényl)-4,5 (cyclopropyl-méthylthio)-2 imidazole.

9. Le bis-(méthoxy-4 phényl)-4,5 (cyclopropylméthylsulfinyl)-2 imidazole.

10. Le bis-(méthoxy-4 phényl)-4,5 (cyclopro-pylméthylsulfonyl)-2 imidazole.

11. Le bis-(méthoxy-4 phényl)-4,5 (cyclopentylméthylthio)-2 imidazole.

12. Le bis-(méthoxy-4 phényl)-4,5 (cyclopentyl-méthylsulfinyl)-2 imidazole.

13. Le bis-(méthoxy-4 phényl)-4,5 (cyclopen-tylméthylsulfonyl)-2 imidazole.

14. Le bis-(méthoxy-4 phényl)-4,5 cyclopen-tylthio-2 imidazole.

15. Le bis-(méthoxy-4 phényl)-4,5 (cyclopen-tylsulfinyl)-2 imidazole.

16. Le bis-(méthoxy-4 phényl)-4,5 (cyclopen-tylsulfonyl)-2 imidazole.

17. Le bis-(méthoxy-4 phényl)-4,5 (hydroxy-2 cyclopentylthio)-2 imidazole.

18. Le bis-(méthoxy-4 phényl)-4,5 (hydroxy-2 cyclopentylsulfinyl)-2 imidazole.

19. Le bis-(méthoxy-4 phényl)-4,5 (hydroxy-2 cyclopentylsulfonyl)-2 imidazole.

20. Le bis-(méthoxy-4 phényl)-4,5 (cyclopro-pylthio)-2 imidazole.

21. Le bis-(méthoxy-4 phényl)-4,5 (cyclopro-pylsulfinyl)-2 imidazole.

22. Le bis-(méthoxy-4 phényl)-4,5 (cyclopro-pylsulfonyl)-2 imidazole.

23. Le bis-(fluoro-4 phényl)-4,5 (cyclopro-pylméthylthio)-2 imidazole.

24. Le bis-(fluoro-4 phényl)-4,5 (cyclopro-pylméthylsulfinyl)-2 imidazole.

25. Le bis-(fluoro-4 phényl)-4,5 (cyclopro-pylméthylsulfonyl)-2 imidazole.

26. Le bis-(méthoxy-2 phényl)-4,5 méthyl-1 (cyclopentylthio)-2 imidazole.

27. Médicaments caractérisés en ce qu'ils contiennent un ou deux dérivés de l'imidazole selon l'une quelconque des revendications 1 à 26.

28. Médicaments selon la revendication 27 pour le traitement de maladies inflammatoires ou allergiques chez l'homme.

29. Procédé de préparation de dérivés de l'imidazole de formule générale I selon la revendication 1, procédé caractérisé en ce que, en opérant de manière connue:

a) on condense un dérivé de l'imidazole répondant à la formule générale II:

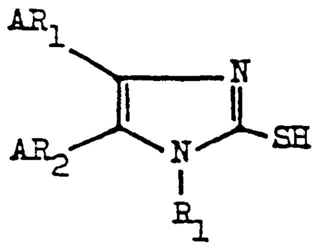

(II)

dans laquelle $AR_1$, $AR_2$ et $R_1$ ont les significations précédemment données, avec un composé répondant à la formule générale III:

WZ (III)

dans laquelle Z a la signification indiquée plus haut et W représente un atome d'halogène, un radical alkylsulfonyloxy ou un radical arylsulfonyloxy, ou

b) lorsqu'on veut préparer des dérivés de l'imidazole de formule générale I dans lesquels Z désigne un radical cycloalkyle, on fait réagir un disulfure de formule générale VII:

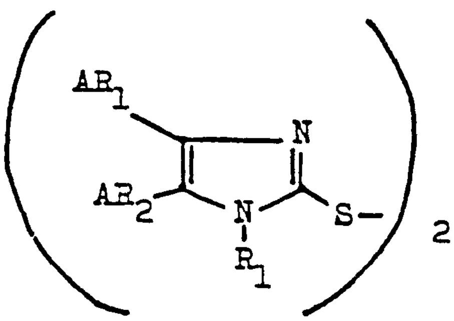

(VII)

avec un cycloalkyl-lithium,

éventuellement on alkyle les dérivés de l'imidazole non substitués en 1 qui ont été obtenus selon l'une ou l'autre des variantes a) et b), on estérifie les dérivés de l'imidazole contenant des radicaux hydroxy, on oxyde des radicaux thio en radicaux sulfinyles ou sulfonyles, et/ou on transforme les dérivés de l'imidazole de formule générale I, par réaction avec des acides acceptables du point de vue physiologique, en sels correspondants.